# EUROPEAN PATENT APPLICATION

(11) **EP 1 880 662 A1**
(43) Date of publication of application: **23.01.2008**
(21) Application number: 07112453.1
(22) Date of filing: 13.07.2007
(51) Int. Cl.: A61B 1/04, A61B 1/005

(54) **Videoguide for medical use in diagnostics and mini-invasive therapeutic treatment**

(30) Priority: 19.07.2006 WO PCT/IT2006/000550
(71) Applicant: Bioampere Research S.r.l., 37100 Verona (IT)
(72) Inventor: Catalano, Ermelinda, 35028, Piove di Sacco (Padova) (IT); Vasina, Lucilla, 25080, Calvagese della Riviera (Brescia) (IT)
(74) Representative: Crippa, Paolo Ernesto

(57) **Abstract**

The present invention relates to a videoguide for medical use in diagnostics and mini-invasive therapeutic treatment, comprising a distal catheter portion suitable for at least partial insertion inside the body of the patient, a proximal tip and at least one optic fibre cable running all along the length of the videoguide.

In the vicinity of the tip of said distal portion an elastic sheath is attached suitable for inflation so as to produce a balloon which surrounds said distal tip.

At least the section of optic fibre cable which runs along the distal portion of the catheter is incorporated into said distal portion so as to form a single body with it.

## Description

Modern medicine has an increasing need to diagnose patients by means of mini-invasive instruments. Mini-invasive instruments are taken to mean instruments of extremely limited dimensions enabling the operator to carry out diagnostic procedures or therapeutic treatment without the need to make large incisions or extensive surgery. One is working therefore with instruments requiring at most incisions of just a few millimetres, or instruments which can be introduced through cannulae (introducers) again with a diameter to the order of several millimetres. Percutaneous techniques are spoken of when the patient is operated on using introducers.

Various percutaneous diagnostic techniques exist. One of these avails of the use of optic fibre cables making it possible to reproduce visible images of the sites explored inside the patient.

The videoguide which the present invention relates to is particularly suitable in epiduroscopy. Epiduroscopy is a percutaneous technique for the diagnosis and treatment of specific diseases of the spinal column. This technique is useful in all those cases where the direct observation of the situation of the spine and eperidural space pernmitted by use of the instrument makes for a clearer understanding of the disorders connected with diseases of the spinal column generating pain.

Using the special introducer kit access is provided to the inside of the column through the sacral hiatus. This consists of a cannula of little more than three millimetres in diameter. This cannula acts as the entryway for access to the eperidural space inside the spinal column.

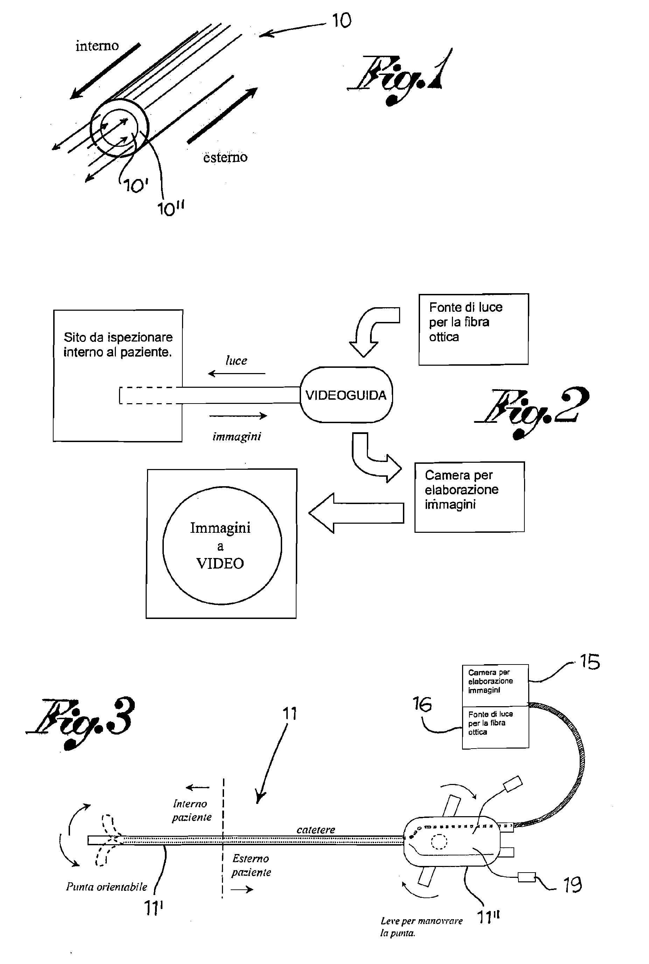

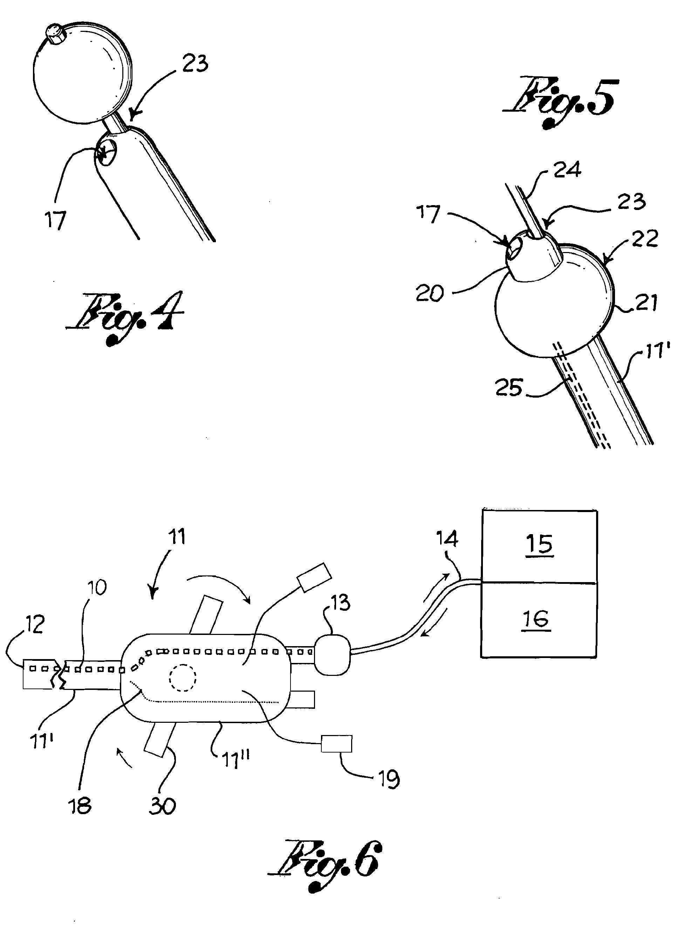

If the inside of the site needs to be observed a system is required which transmits the picture as well as a light source to illuminate the inside of the space examined. These two functions are both absolved by flexible optic fibre cables which in one section of the cable, generally the outer section, are used to transmit light to the tip, while the rest of the inner part has the opposite function of transmitting the images from the inside towards the outside of the site examined (fig. 1). This latter visual signal is conveyed, by means of external cables, to a camera which processes and reproduces the video image (fig. 2).

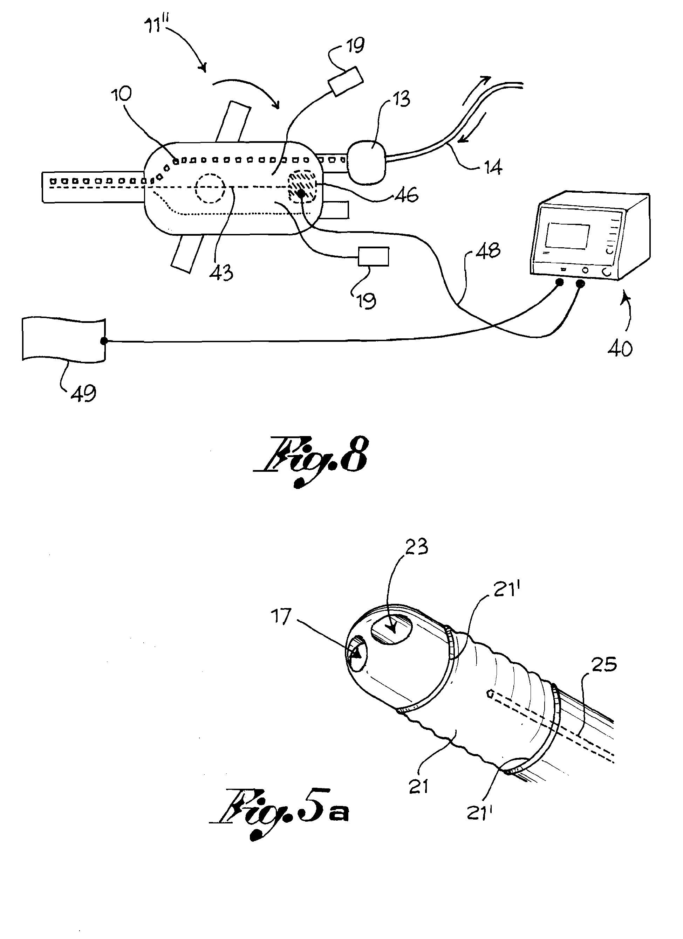

The introduction of the optic fibre cable inside the patient's body is not subordinate to the use of introducers alone, but also to the use of a support system making it possible to direct the field of vision of the fibre in the direction wished by means of the movements made to it from the outside. This latter object is called a "videoguide" (fig. 3). In other words, the videoguide is an instrument which makes it possible, by means of the external hand grip, to flex the extremity of the catheter which is inside the patient's body. The directable catheter of the videoguide contains the optic fibre: this way it is possible to direct both the beam of light and the area being filmed inside the eperidural space by means of external manoeuvres by the operator. The optic fibre examination can, with these movements of the tip, thus be extended to areas outside the main area, without damaging the internal tissues of the patient or the optic fibre itself.

As one might imagine all the instrumentation coming into contact with the patient during performance of the operation must necessarily be sterile.

Among the various instruments therefore, a sterile videoguide and sterile optic fibre must be available.

As regards the videoguide, various models exist for sale, both disposable versions and those designed for repeated use (therefore requiring sterilisation after each application). In epiduroscopy only disposable videoguides are used.

The use of videoguides which can be re-sterilised is reserved for medical applications of lesser importance.

As regards the optic fibre however, currently it is standard practice to use a model designed for repeated use which can therefore be re-sterilised after each application.

In current conditions optic fibres are always of a length of a metre and with a diameter of roughly a millimetre. Being made of fibreglass, special care must be taken when handling this cable requires and after a relatively small number of sterilisation cycles, the optic fibre cable is generally damaged to the point that it requires replacement. These cables with these characteristics are extremely expensive, to the extent that the purchase of new cables is a considerably limiting factor for this technique.

Another important point to emphasise relates to the sterilisation process which generally takes several hours. In logistic terms this constitutes another significant limitation: for an operating session using the epiduroscopy method with a number of patients, the same number of optic fibre cables as patients will be needed since the time needed for re-sterilisation is prohibitive. As a result what happens in many centres is that this sort of technique is reserved to not more than two or three patients at a time.

As already said, the videoguide is fitted with two operating channels: one is always occupied by the optic fibre needed to provide visibility in the site being examined while the other is reserved for the use of accessories which, depending on the clinical situation, enable the operator to treat diseased areas. These accessory instruments vary as regards use and function; the only characteristics they have in common are the maximum diameter which must obviously not exceed that of the operating channel through which they must pass, and their flexibility, given that, as described above, the tip of the videoguide is directable and manoeuvrable by means of functions controlled directly from the outside by the operator.

One of the accessories is the so-called balloon or more correctly Fogarty catheter after its inventor. This particular type of catheter is a sort of flexible tube closed at one end. The other open end is fitted with a "lower look" type attachment where the syringe is attached. The distinguishing feature of the catheter is that the closed end is made from biocompatible elastic material for a length varying depending on the model. For epiduroscopic use the length of this section does not generally exceed one centimetre.

When pressure is exerted inside the catheter, such as for example when injecting physiological solution using the syringe from the open end, the closed end of the catheter is deformed in the part made from elastic material. A balloon is thus formed at the tip of the catheter which inflates depending on the pressure exerted from the outside.

The function of this instrument is merely mechanical. In surgical practice and especially epiduroscopy, the use of the Fogarty catheter is indicated in all those cases in which organic tissues which for whatever reason have become entangled creating abnormal clinical conditions, need to be distanced, distended or separated. In epiduroscopy it is often the case that the tissues of the dural roof are "stuck" to the dura madre where the catheter of the videoguide sits.

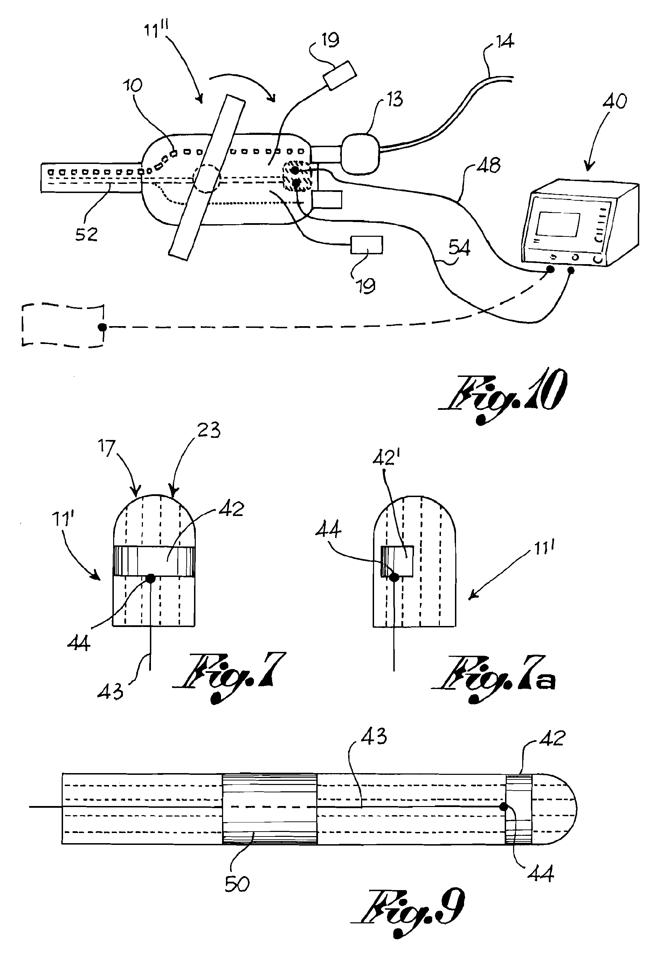

In conclusion, the tip of the videoguide is inserted between the various tissues stuck to each other, then with the introduction of the Fogarty into the operating channel the tip is made to protrude sufficiently so as to allow the balloon to inflate and "separate" the treated areas. This operation also makes it possible to break all the strangulating fibres which bind the roots in a pathological manner, generating pain (fig. 4).

The use of the Fogarty entails some difficulties however.

While the balloon is dilated the field of vision is temporarily occluded.

The small size of the balloon, dictated by the need to pass the catheter through the operating channel, is sometimes of limited effect. If then, one exceeds in the dilation of the balloon it may burst.

The operating channel is occupied by the Fogarty. It is not possible to use other instruments at the same time as the balloon. In fact with one free operating channel only one instrument can be used at a time.

The object of the present invention is to offer a videoguide able to overcome the problems mentioned above.

Such aim is achieved by a videoguide conforming to the following claims.

Further details and advantages of the videoguide according to the present invention will appear more clearly from the following description of preferred embodiments thereof, made by way of an indicative and non-limiting example given with reference to the annexed figures, wherein:

Figure 1 shows an optic fibre used in a videoguide

Figure 2 is a flow diagram of an inspection system using a videoguide;

Figure 3 shows the videoguide only in a schematic manner;

Figure 4 is a view of the distal end of a state of the art videoguide, with the balloon under pressure;

Figure 5 is a view of the distal end of the videoguide according to the present invention, with the balloon under pressure;

Figure 5a is a view of the distal end of the videoguide according to the present invention, with the balloon not under pressure;

Figure 6 is a schematic view of the proximal end of the videoguide according to the present invention;

Figure 7 is a schematic illustration of the distal end of the videoguide fitted with an electrode for radiofrequency treatment;

Figure 7a is a similar view to the previous with the electrode in another embodiment;

Figure 8 is a diagram of the electrical connections of the videoguide with electrode to a radiofrequency field generator;

Figure 9 is a schematic illustration of the distal portion of a videoguide fitted with two electrodes for "bipolar" radiofrequency treatment; and

Figure 10 is a diagram of the electrical connections of the videoguide of figure 9 to a radiofrequency generator;

With reference to figure 6 specifically, one embodiment of the device according to the invention provides the combination of a disposable optic fibre 10 with a videoguide 11.

The grafting of a section of disposable optic fibre 10 directly onto the catheter of the videoguide 11 is technically possible. For example, the catheter is produced by extrusion and subsequently the section 10 of optic fibre is attached inside the operating channel of the catheter set aside for it using fastening systems, such as special glues. The fibre is fixed so that its distal end 12 is aligned with that of the videoguide 11.

The "integrated videoguide - optic catheter" system remains flexible and directable by means of external manoeuvring by the operator so that from a technical point of view the method of examination remains unchanged and no new difficulties are encountered in handling the system. The advantage of a disposable videoguide system which also includes the optic fibre section is enormous. In fact, with traditional systems the videoguide is always disposable and the optic fibre re-sterilisable. This situation entails the re-sterilisation of the optic fibre after each application, with the consequent difficulties described above.

More in detail the sterile videoguide 11 has the section of optic fibre 10 which extends all along the length of the videoguide (approx. 60cm) incorporated in the catheter.

According to a preferred embodiment, the optic fibre 10 is of the type shown in figure 1. It includes an inner nucleus 10' able to transmit an image from the inside to the outside of the site under examination and an external sheath 10" able to transmit the light from the outside to the tip of the cable. The image is sent to a camera 15 which processes it and reproduces it in video (fig. 2). The light comes from an external light source 16.

In the proximal tip of the videoguide 11 an attachment system 13 is fitted, of the bayonet or "lower look" type, where an optic coupling is made between the section of fibre 10 incorporated into the videoguide and the remaining cable 14 which connects to the camera 15 for the reproduction of video images and to the light source 16.

In other words, and with reference for example to figures 3 and 6, the videoguide 11 includes a distal catheter portion 11' suitable for insertion at least in part, in the patient's body, a proximal tip 11", and at least one optic fibre cable 10, 14 which extends all along the length of the videoguide and which protrudes from the said proximal tip to be connected to image processing devices 15 and a light source 16. According to the invention, at least the section of optic fibre cable 10 which extends along the distal catheter portion 11' is incorporated with the said distal portion so as to be one with it.

Advantageously, the entire section of optic fibre cable extending from the distal tip to the proximal tip of the videoguide is one with the videoguide.

According to a preferred embodiment, at the proximal tip 11" of the videoguide there is a releasable optic connection and coupling 13 between the said section of cable 10 incorporated with the videoguide and a repeated use optic fibre cable 14 coming from the said image processing device 15 and the said light source 16.

According to one embodiment, said section of incorporated optic fibre cable 10 is housed in a permanent manner in its respective operating channel 17 made in the videoguide 11.

Advantageously, the videoguide 11 includes at least another operating channel 18 for the introduction of at least one accessory instrument (figure 6).

It should be noted that the proximal tip 11" of the videoguide 11 is fitted with means of manoeuvring 30 able to direct the distal tip of the distal catheter portion 11'.

Furthermore, the videoguide 11 is provided with at least one infusion entry point 19.

The key point of this system is the fact that it is no longer necessary to re-sterilise the optic fibre cable 14 which, remaining outside the patient at all times no longer constitutes a risk of contamination for the operating field. In fact, the part which must be sterile is the section of disposable fibre 10 incorporated in the videoguide and this is connected to by means of the external attachment 13 provided at the terminal of the videoguide.

The optic coupling between the two fibre sections 10, 14 does not introduce any phenomena of signal alteration which is in any case processed and transmitted to the video. It should also be borne in mind that with traditional systems there is in any case an interruption of the cable before entering the camera for processing of the signal (in fact the cable currently has to be disconnected from the camera so as to be re-sterilised). As a result, the use of a section of disposable optic fibre merely involves moving this optic coupling from upline to downline of the entire system. In any case, even the introduction of a further coupling system in the transmission of the signal through the fibre would not entail difficulties related to the definition of the image.

According to another aspect of the invention, at the distal tip 20 of the catheter of the videoguide 11 an elastic sheath 21 is applied to give the videoguide a "Fogarty" type function.

According to one embodiment, the elastic sheath 21 is in the form of an elastic collar, in silicon or derivatives for example, which is slipped on to the distal tip 11' of the videoguide 11 and attached to it at the edges 21', with a chemical sealant for example. Alternatively the collar 21 may be mechanically attached by means of clips or ties.

The functions of being directable and accessible through two operating channels are maintained; in addition, there is the possibility of inflating a balloon 22 on the distal tip 20 of the videoguide thus achieving the great advantage of keeping the operating channel 23 free, which in the traditional method was occupied by the Fogarty. The operating channel 23 can thus be used to insert an accessory instrument 24.

The other operating channel 17 illustrated in detail in figures 5 and 5a is the one used for the optic fibre cable 10.

The balloon 22 is generally inflated and filled with injections of physiological solution from the outside. To make this possible, a lumen 25 is made in the videoguide 11 specifically for this function (figure 5). Said lumen 25 is directly connected to one of the infusion entry points 19 present externally to which a syringe is connected with which to inject the physiological solution. Depending on the pressure exerted the dimensions of the balloon may be varied.

This integrated system thus enables the contemporary use of the balloon 22 with another accessory 24 such as a grasper or radiofrequency probe for example.

Another advantage is the fact that the balloon 22, when inflated, does not occupy the field of vision of the optic fibre. On the contrary, by dilating the tissues, it facilitates the use of accessories which are used at the same time, since the radius of action within the epidural space gives a physically larger area of operation.

According to a further embodiment, the videoguide according to the present invention can be electrically wired to a radiofrequency generator 40 for neuro-modulating treatment of nervous tissue. For example, it's possible to operate on epidural nervous tissue with pulsed radiofrequency treatment. The videoguide 11 is thus fitted with appropriate electrical contacts and connections.

According to one embodiment for "monopolar" treatment, an electrode 42 is attached to the distal portion 11' of the videoguide 11 by means of which a radiofrequency field is generated inside the site to be treated. In an example of an embodiment illustrated schematically in figure 7, the electrode 42 is in the form of a ring positioned just above the curvature of the distal tip 20 of the videoguide. The electrode 42 is for example a cylindrical shape coaxial to the videoguide and may be completely exposed or only partially exposed, so as to limit the metallic surface in contact with the tissue.

The same electrode 42 may be made, rather than with a coaxial ring, with a metallic plate 42' grafted onto the body of the catheter, as shown schematically in figure 7a. The choice of electrode, in shape and size, depends on manufacturing criteria and varies in relation to the chosen dimensions of the extrusion of the catheter forming the videoguide.

Furthermore, advantageously, given that the generators 40 which are used for radiofrequency procedures usually control the power supply according to the monitored temperature of the tissue being treated, the videoguide has a thermocouple 44 to measure the temperature. The thermocouple 44 is situated next to the electrode 42 and is connected to the generator 40.

Figure 8 provides a schematic diagram of how the videoguide with electrode 40 is connected to the generator 40 for monopolar treatment. The electrode 42, 42' and the thermocouple 44 if present, are connected by means of electric conductors 43 which run along inside the catheter casing of the videoguide, to a corresponding electric connector 46 (for example of the female type) at the proximal tip 11" of the videoguide. Said connector 46 is suitable for coupling to a complementary electric connector (for example of the male type) situated at the tip of an electric cable 48 connected to the generator 40.

The presence of a ground plate 49 which can be attached to a remote part of the patient so as to close the electric circuit can also be observed.

In an embodiment variation suitable for "bipolar" type treatment, the electric circuit is closed by a second electrode 50 situated close to the first electrode 42, 42' (figure 9), and therefore inside the site being treated.

The second electrode 50 has an exposed surface area comparable to the first. Preferably, for safety reasons, the second electrode 50 is a slightly larger size than the first, where the temperature is measured by means of the thermocouple 44, this way the highest temperature in the area treated remains in any case that measured in the first electrode (in effect fitted with a thermocouple for temperature monitoring), because it is smaller and therefore has a greater density of current.

The second electrode 50 is coaxial to the catheter and in a more proximal position, distanced for example 2-3cm from the first electrode 42.

Figure 10 provides a schematic diagram of the electric wiring of the videoguide 11 for bipolar treatment. The presence of a second electric conductor 52 which connects the second electrode 50 to a corresponding connector at the proximal tip 11" of the videoguide 11 may be observed, said connector being suitable for coupling to a respective electric cable 54 connected to the generator 40.

It may also be observed in this case that the use of the ground plate 49 is no longer necessary.

Obviously, a skilled person may make further modifications and variations to the videoguide according to the present invention so as to satisfy contingent and specific needs, all of which moreover contained within the scope of protection of the invention, as defined by the following claims.

## Claims

1. Videoguide (11) for medical use in diagnostics and mini-invasive therapeutic treatment, comprising a distal catheter portion (11') suitable for at least partial insertion inside the body of the patient, an optic fibre (10) being associable with such distal portion, **characterised by** the fact that in the vicinity of the distal tip (20) of said distal portion (11') an elastic sheath (21) suitable to be inflated so as to obtain a balloon (22) which surrounds said distal tip (20).

2. Videoguide according to claim 1, wherein said elastic sheath (21) is in fluidic communication with a lumen (25) made in the videoguide and able to take in fluid so as to inflate said sheath.

3. Videoguide according to claim 2, wherein said lumen is in fluidic communication with a corresponding infusion entry point (19) at the proximal tip of the videoguide.

4. Videoguide according to any of the preceding claims, comprising at least one optic fibre cable (10) which runs all along the length of the videoguide and which protrudes from said proximal tip for connection to image processing devices (15) and to a light source (16), **characterised by** the fact that at least the section of optic fibre cable which runs along the distal portion of the catheter is incorporated into said distal portion so as to form a single body with it.

5. Videoguide according to claim 4, wherein all the section of optic fibre cable which runs from the distal tip to the proximal tip of the videoguide forms a single body with the videoguide.

6. Videoguide according to claim 5, wherein at the proximal tip of the videoguide there is a releasable system of optic coupling and connection (13) between said section of cable incorporated into the videoguide and a repeated use optic fibre cable coming from said image processing devices and from said light source.

7. Videoguide according to any of the preceding claims, wherein said section of incorporated optic fibre cable (10) is attached in a permanent manner in its respective operating channel (17) made in the videoguide.

8. Videoguide according to any of the preceding claims, comprising at least one further operating channel (18; 23) for the introduction of at least one accessory instrument (24).

9. Videoguide according to any of the preceding claims, wherein the proximal extremity has a corresponding means of manoeuvring (30) able to direct the distal tip of the distal catheter portion.

10. Videoguide for medical use in diagnostics and mini-invasive therapeutic treatment, comprising a distal catheter portion suitable for at least partial insertion inside the body of the patient **characterised by** the fact that in the vicinity of the tip of said distal portion an elastic sheath is attached which can be inflated so as to produce a balloon surrounding said distal tip.

11. Videoguide according to any of the preceding claims, wherein said elastic sheath is in the form of an elastic collar fitted onto the distal tip (11') of the videoguide (11) and attached to it at the edges (21').

12. Videoguide according to claim 11, wherein the elastic collar is attached to the videoguide by means of chemical sealant.

13. Videoguide according to claim 11, wherein the elastic collar is attached to the videoguide mechanically by means of ties or clips.

14. Videoguide for medical use in diagnostics and mini-invasive therapeutic treatment, comprising a distal catheter portion suitable for at least partial insertion inside the body of the patient, a proximal tip and at least one optic fibre cable running all along the length of the videoguide and protruding from said proximal tip for connection to image processing devices and a light source, **characterised by** the fact that at least the section of optic fibre cable which runs along the distal portion of the catheter is incorporated in such distal portion so as to form a single body with it.

15. Videoguide according to any of the preceding claims, wherein the distal portion (11') of the videoguide (11) is attached at least to the first electrode (42; 42') which can be connected to a generator of a radiofrequency field (40).

16. Videoguide according to claim 15, wherein a thermocouple (44) is associated with said first electrode (42).

17. Videoguide according to claims 15 or 16, wherein upline and in the vicinity of the first electrode (42) a second electrode (50) is provided which can be electrically connected to the radio frequency generator (40) to close the electric circuit in the case of "bipolar" treatment.
